# EUROPEAN PATENT SPECIFICATION

(11) **EP 2 119 367 B1**
(45) Date of publication and mention of the grant of the patent: **21.08.2013**
(21) Application number: 07851059.1
(22) Date of filing: 21.12.2007
(51) Int. Cl.: A23D 9/00, A23K 1/16, A23L 1/24, A23L 1/30, A23L 2/52

(54) **OIL-AND-FAT COMPOSITION, AND FOOD OR BEVERAGE CONTAINING THE OIL-AND-FAT COMPOSITION**
ÖL-UND-FETT-ZUSAMMENSETZUNG SOWIE NAHRUNGSMITTEL ODER GETRÄNK, DAS DIE ÖL-UND-FETT-ZUSAMMENSETZUNG ENTHÄLT
COMPOSITION HUILE ET GRAISSE, ET ALIMENT OU BOISSON CONTENANT LA COMPOSITION HUILE ET GRAISSE

(30) Priority: 28.12.2006 JP 2006356388
(43) Date of publication of application: 18.11.2009
(73) Proprietor: The Nisshin OilliO Group, Ltd., Tokyo 104-8285 (JP)
(72) Inventor: NODA, Ryuuji, Yokosuka-shi Kanagawa 239-0832 (JP); SEKINE, Seiji, Yokosuka-shi Kanagawa 239-0832 (JP); OGASAWARA, Chika, Yokosuka-shi Kanagawa 239-0832 (JP); TAKEUCHI, Hiroyuki, Yokosuka-shi Kanagawa 239-0832 (JP); IKUINA, Junichi, Yokosuka-shi Kanagawa 239-0832 (JP); TOYOSHIMA, Takashi, Yokosuka-shi Kanagawa 239-0832 (JP)
(74) Representative: Denison, Christopher Marcus
(86) International application number: PCT/JP2007/074707
(87) International publication number: WO 2008/081754

(56) References cited:
- WO-A1-97/28695
- WO-A1-2006/052974
- JP-A- 63 036 744
- JP-A- 2000 157 170
- JP-A- 2003 079 314
- JP-A- 2004 254 588
- US-A1- 2005 054 724
- DATABASE WPI Week 200031 Thomson Scientific, London, GB; AN 2000-353657 XP002662007, & JP 2000 109882 A (NIPPON OILS & FATS CO LTD) 18 April 2000 (2000-04-18)
- DJOUSSE: "Dietary Linolenic Acid Is Associated With a Lower Prevalence of Hypertension in the NHLBI Family Heart Study", HYPERTENSION, vol. 45, 1 January 2005 (2005-01-01), pages 368-373, XP55009948, ISSN: 0194-911X
- HIROYUKI TAKEUCHI ET AL: "Antihypertensive Effect and Safety of Dietary [alpha]-Linolenic Acid in Subjects with High-Normal Blood Pressure and Mild Hypertension", JOURNAL OF OLEO SCIENCE, vol. 56, no. 7, 5 June 2007 (2007-06-05), pages 347-360, XP55010211, ISSN: 1345-8957
- HUNTER: "n-3 fatty acids from vegetable oils.", AMERICAN JOURNAL OF CLINICAL NUTRITION, vol. 51, no. 5, 1 May 1990 (1990-05-01), pages 809-814, XP55010341, ISSN: 0002-9165

## Description

### FIELD OF THE INVENTION

This invention relates to an oil-and-fat composition having a high α-linolenic acid content and a food or beverage containing the oil-and-fat composition.

### BACKGROUND ART

α-linolenic acid is a kind of n-**3** polyunsaturated fatty acid and is called as an essential fatty acid, but it can not be synthesized in vivo, so that it should be consumed in foods.

Some of the vegetable oils have the α-linolenic acid content of not less than **50** mass% in the constituent fatty acids, and as the typical examples, flax oil (flaxseed oil), perilla oil (egoma oil) and perilla seed oil (shiso seed oil) can be given.

However, the oils having the α-linolenic acid content of not less than **50** mass% such as flax oil, perilla oil and perilla seed oil are easily deteriorated and have the problems of flavor and storage stability, therefore, it was difficult to realize the product commercialization. Further, in a manufacturing process of the vegetable oil, normally, a deodorization process is carried out, however, α-linolenic acid has a structure that tree double bonds are included, and due to this, it has also a problem that it is easily turned into trans fatty acid.

As a countermeasure for solving the above-mentioned problem, an edible oil is disclosed, which has the α-linolenic acid content of not less than **10** mass% obtained by adding a high-oleic oil-and-fat having the oleic acid content of not less than **60** mass% to flax oil having the α-linolenic acid content of not less than **50** mass% and the trans fatty acid content of not more than **4** mass%, and the ratio by mass of the oleic acid to the α-linolenic acid is controlled so as to be **1.0 : 0.2-0.6.** (refer to Patent Literature **1**)

Patent Literature **1** discloses that flax oil can be manufactured, which has a good flavor and the trans fatty acid content suppressed to not more than **4** mass%, if the deodorization process in the manufacturing process of flax oil is carried out in the condition that the deodorization temperature is **220** to **240** degrees C, the deodorization time is **60** to **90** minutes and the blowing amount of steam to the oil is **3.5** to **5.5** mass%.

Further, an oil-and-fat composition containing α-linolenic acid is disclosed, which includes rice oil having an odor masking effect (refer to Patent Literatures **2, 3**).

Patent Literature **2** discloses an oil-and-fat composition comprising **30** to **60** weight% of an edible oil-and-fat derived from rice and **5** to **10** weight% of an oil-and-fat having α-linolenic acid content of not less than **45** weight%, and Literature **3** discloses an oil-and-fat composition comprising not more than **10** weight% of an oil-and-fat having α-linolenic acid content of not less than **50** weight%, and more than **60** weight% and not more than **85** weight% of an edible oil-and-fat derived from rice, of the whole oil-and-fat composition.

On the other hand, it is reported that α-linolenic acid plenteously contained in flax oil can decrease a risk of development of hypertension in human epidemiological study (refer to Non Patent Literature **1**). Further, it is known that an edible oil-and-fat composition having a high α-linolenic acid content has a preventive effect against the hypertension (refer to Patent Literature **4**).
Patent Literature **1:** JP-A-2000-157170
Patent Literature **2:** JP-A-2003-79314
Patent Literature **3:** JP-A-2004-254588
Patent Literature **4:** JP-A- S63 (1988)-36744

Non Patent Literature **1:** Luc Djousse et al. "Dietary Linolenic Acid Is Associated With a Lower Prevalence of Hypertension in the NHLBI Family Heart Study." Hypertension, volume 45, p. 368 to 373, 2005

### DISCLOSURE OF THE INVENTION

### PROBLEMS TO BE SOLVED BY THE INVENTION

However, as previously mentioned, the oils having the α-linolenic acid content of not less than 50 % such as flax oil, perilla oil and perilla seed oil have the problems of flavor and storage stability, therefore, if the oil-and-fat composition is configured to have a high α-linolenic acid content so as to achieve the preventive effect against the hypertension, it becomes difficult to obtain the oil-and-fat composition having a good flavor, and good storage stability, even if the edible oils described in Patent Literatures **1** to **3** are used.

Therefore, it is an object of the invention to provide an oil-and-fat composition having a high α-linolenic acid content, a hypertension-ameliorating effect, a good flavor, and good storage stability, and a food or beverage containing the oil-and-fat composition.

### MEANS FOR SOLVING THE PROBLEMS

In order to achieve the above-mentioned object, the invention provides an oil-and-fat composition, comprising: at least one vegetable oil A selected from flax oil, perilla oil and perilla seed oil; rice oil; and at least one vegetable oil B selected from rape seed oil, soybean oil and corn oil, at a ratio among the vegetable oil A, the rice oil and the vegetable oil B of **20-35/15-25/40-65** by mass, wherein; the α-linolenic acid content in the constituent fatty acids contained in the oil-and-fat composition is **15** to **30** mass%.

Further, in order to achieve the above-mentioned object, the invention provides an oil-and-fat composition, comprising: **20-35** mass% of at least one vegetable oil A selected from flax oil, perilla oil and perilla seed oil; and **15-25** mass% of rice oil, wherein; the α-linolenic acid content in the constituent fatty acids contained in the oil-and-fat composition is **15** to **30** mass%.

Further, in order to achieve the above-mentioned object, the invention provides an oil-and-fat composition, comprising: **30-53** mass% of at least one vegetable oil A selected from flax oil, perilla oil and perilla seed oil; and **70-47** mass% of rice oil, wherein; the α-linolenic acid content in the constituent fatty acids contained in the oil-and-fat composition is **15** to **30** mass%.

Further, in order to achieve the above-mentioned object, the invention provides a food or beverage containing the oil-and-fat composition according to the inventions.

### ADVANTAGES OF THE INVENTION

According to the invention, an oil-and-fat composition having a high α-linolenic acid content, a hypertension-ameliorating effect, a good flavor, and good storage stability, and a food or beverage containing the oil-and-fat composition can be provided.

### BEST MODE FOR CARRYING OUT THE INVENTION

### [Oil-and-fat composition according to a first embodiment of the invention]

An oil-and-fat composition according to a first embodiment of the Invention comprises at least one vegetable oil A selected from flax oil, perilla oil and perilla seed oil; rice oil; and at least one vegetable oil B selected from rape seed oil, soybean oil and corn oil, at a ratio among the vegetable oil A, the rice oil and the vegetable oil B of **20-35/15-25/40-65** by mass, wherein; the α-linolenic acid content in the constituent fatty acids contained in the oil-and-fat composition is **15** to **30** mass%.

### (Vegetable oil A)

The oil-and-fat composition according to the first embodiment includes at least one vegetable oil selected from flax oil, perilla oil and perilla seed oil. Particularly, it is preferable to use flax oil, and more preferable to use the flax oil having the α-linolenic acid content of not less than **50**%, and the trans fatty acid content in the α-linolenic acid of not more than **4** mass%. It is preferable that the trans fatty acid content is decreased as much as possible, since the trans fatty acid is not nutritionally preferable, for example, if it is consumed, an essential fatty acid requirement in vivo is increased.

### (Flax oil)

Flax oil is obtained by pressing flax seeds, and has the α-linolenic acid content of not less than about **50** mass% in the constituent fatty acids, so that it can be preferably used in the embodiment. In the embodiment, particularly, it is preferable to use flax oil having the α-linolenic acid content of not less than **50** mass% in the constituent fatty acids, and the trans fatty acid content in the α-linolenic acid of not more than **4** mass%, for example, it can be manufactured by the following manufacturing condition.

That is to say, the flax oil can be obtained, which has the α-linolenic acid content of not less than **50** mass% in the constituent fatty acids, and the trans fatty acid content of not more than **4** mass%, if the deodorization process in the manufacturing process of flax oil is carried out in the condition that the deodorization temperature is **220** to **240** degrees C, the deodorization time is **30** to **90** minutes, preferably **50** to **90** minutes and the blowing amount of steam to the oil is **2.0** to **5.5** mass%, preferably **3.0** to **4.5** mass%.

### (Rice oil)

The oil-and-fat composition according to the embodiment includes rice oil. If rice oil is blended together with the vegetable oil A such as flax oil, it can enhance a cooking aptitude, a flavor, and an oxidation stability by compensating the weakness of the vegetable oil A such as flax oil. As the rice oil, distilled rice oil to which alkaki deacidification treatment is not applied can be preferably used, but it is more preferable to use the rice oil to which alkaki deacidification treatment is applied in terms of crystallinity and the like.

### (Vegetable oil B)

The oil-and-fat composition according to the embodiment includes at least one vegetable oil selected from rape seed oil, soybean oil and corn oil. Particularly, it is preferable to use rape seed oil. If the vegetable oil B is blended together with the vegetable oil A such as flax oil, it can enhance a cooking aptitude, a flavor, and an oxidation stability by compensating the weakness of the vegetable oil A such as flax oil.

### (Blending ratio in oil-and-fat composition)

The oil-and-fat composition according to the embodiment includes the vegetable oil A, rice oil and the vegetable oil B at a ratio among the vegetable oil A, the rice oil and the vegetable oil B of **20-35/15-25/40-65** by mass. That is to say, if the other components are not blended, the oil-and-fat composition according to the embodiment includes **20-35** mass% of the vegetable oil A, **15-25** mass% of the rice oil and **40-65** mass% of the vegetable oil B. If the vegetable oil A, the rice oil and the vegetable oil B are blended at the above-mentioned blending ratio, the oil-and-fat composition having a hypertension-ameliorating effect, a good flavor, and good storage stability can be obtained. The oil-and-fat composition according to the embodiment can be prepared by that the respective oil-and-fats stored in the respective tanks are mixed and stirred in a tank for blending.

### (α-linolenic acid content)

**In t**he oil-and-fat composition according to the Embodiment, the α-linolenic acid content in the constituent fatty acids contained in the oil-and-fat composition is **15** to **30** mass%. If less than **15** mass%, a hypertension-ameliorating effect can not be highly expected, and if more than **30** mass%, a problem of a flavor is caused.

### (Trans fatty acid content in oil-and-fat composition)

The oil-and-fat composition according to the Embodiment has the trans fatty acid content of preferably not more than **3** mass%, and more preferably not more than **2** mass% in the oil-and-fat composition.

### (Other blending components)

Components to be contained in the oil-and-fat composition according to the embodiment other than the vegetable oil A, the rice oil and the vegetable oil B include sesame oil, sesame salad oil, peanut oil, high-oleic safflower oil, high-linoleic safflower oil, sunflower oil, high-oleic sunflower oil, mid-oleic sunflower oil, high-linoleic sunflower oil, cotton seed oil, grape seed oil, macadamia nuts oil, hazelnut oil, pumpkin seed oil, walnut oil, camellia oil, tea seed oil, borage oil, olive oil, rice bran oil, wheat germ oil, medium chain fatty acid-containing oil, palm oil, palm kernel oil, copra oil, cacao butter, oil-and-fat low saturated by breed improvement, beef fat, lard, chicken fat, milk fat, fish oil, seal oil, alga oil and the like, at least one of them can be used. Further, hydrogenated, fractionated, and ester exchanged oil-and-fat thereof can be also used. Further, emulsifying agent such as monoglycerin fatty acid ester, organic acid monoglycerin fatty acid ester, polyglyceryl fatty acid ester, sucrose fatty acid ester, sorbitan fatty acid ester; ascorbic acid fatty acid ester; lignan; coenzyme Q; phospholipid; orizanol; vitamin E; phytosterol; diglyceride; catechins; and polyphenols can be added. Furthermore, carbohydrate, protein, dietary fiber, vitamin, mineral and the like can be also added.

### (Use of oil-and-fat composition and food or beverage containing oil-and-fat composition)

The oil-and-fat composition according to the embodiment can be used for deep-fried food (tempura), stir-frying oil, mold-releasing oil and the like as well as conventional edible oil. A food or beverage containing the oil-and-fat composition according to the embodiment is not particularly limited, and includes food, processed food, health food, beverage, health drink, seasoning, confectionery, pet food, and animals and plants feeding stuff, in particular, gelatin capsule, edible oil, dressing, margarine, prepared margarine, fat spread, cream, ice cream, mayonnaise, bread, cake, doughnut, muffin, scone, fried food, snack food, and diet fluid.

The oil-and-fat composition according to the invention is consumed in an amount of preferably **0.1** g to **20** g, preferably **0.7** g to **14** g, more preferably **1.5** g to **9** g, most preferably **1.8** g to **6** g in adults as an amount of α-linolenic acid per day.

The oil-and-fat composition content in a food or beverage varies according to species of the food or beverage, but the oil-and-fat composition can be contained in the food or beverage in an amount of **0.5**% to **100**%, and it can be directly used for deep-fried food (tempura), stir-frying oil, mold-releasing oil and the like. The oil-and-fat composition is preferably contained in the food or beverage in an amount of, for example, **0.1** g to **20** g in **100** g of dressing, **7.5** g to **24** g in **100** g of mayonnaise, **6** g to **29.4** g in **100** g of water-in-oil type composition as represented by margarine and fat spread, **0.5** g to **9** g in **100** g of bread, **0.6** g to **7.5** g in **100** g of deep-fried product such as deep-fried food(tempura) and fried food, as an amount of α-linolenic acid.

### (Container)

A container of the oil-and-fat composition according to the embodiment includes, for example, can, glass bottle, plastic or paper product, which are heretofore known.

### [Advantages of first embodiment of the invention]

According to the embodiment of the invention, the following advantages are provided.
**(1)** An edible oil having a high α-linolenic acid content, a hypertension-ameliorating effect, a good flavor, and good storage stability can be obtained. Here, the hypertension-ameliorating effect means an effect that higher blood pressure than normal blood pressure is lowered so as to be approximated to the normal blood pressure, or to be improved to the normal blood pressure or an optimum blood pressure. The normal blood pressure means that systolic blood pressure is in the range of not less than **120** mmHg and less than **130** mmHg and diastolic blood pressure is in the range of not less than **80** mmHg and less than **85** mmHg, and the optimum blood pressure means that systolic blood pressure is less than **120** mmHg and diastolic blood pressure is less than **80** mmHg.
**(2)** An edible oil having an excellent aptitude for a cooking (having a good odor at the cooking and a good flavor at the eating) can be obtained, even though it has a high α-linolenic acid content.
**(3)** A food or beverage containing the edible oil having the above-mentioned effect can be obtained.

### [Oil-and-fat composition according to a second embodiment of the invention]

An oil-and-fat composition according to a second embodiment of the invention comprises **20-35** mass% of at least one vegetable oil A selected from flax oil, perilla oil and perilla seed oil, and **15-25** mass% of rice oil, wherein the α-linolenic acid content in the constituent fatty acids contained in the oil-and-fat composition is **15** to **30** mass%.

The present embodiment is different from the above-mentioned first embodiment in that it is not indispensable to blend the vegetable oil B, but it is similar to the above-mentioned first embodiment in the other features.

### [Oil-and-fat composition according to a third embodiment of the invention]

An oil-and-fat composition according to a third embodiment of the invention comprises **30-53** mass% of at least one vegetable oil A selected from flax oil, perilla oil and perilla seed oil and **70-47** mass% of rice oil, wherein the α-linolenic acid content in the constituent fatty acids contained in the oil-and-fat composition is **15** to **30** mass%.

The present embodiment is different from the above-mentioned first embodiment in that the blending of the vegetable oil B is not indispensable, and the ranges of the blending ratio of the vegetable oil A and rice oil are different respectively, but it is similar to the above-mentioned first embodiment in the other features.

### EXAMPLE

Hereinafter, the present invention will be explained by Examples in further detail, but the present invention is not limited by them.

### (Examples 1 to 9 and Comparative Examples 1 to 4)

Oil-and-fat compositions of Examples **1** to **9** and Comparative Examples **1** to **4** were fabricated at the blending ratios described in Tables **1** to **3** by using flax oil (manufactured by the Nisshin OilliO Group Ltd. by using the method described below), rice oil (manufactured by the Nisshin OilliO Group Ltd. and sold under a trade name of "Nisshin rice salad oil"), rape seed salad oil (manufactured by the Nisshin OilliO Group Ltd. and sold under a trade name of "Nisshin rape seed salad oil (S)"), high-oleic safflower oil (manufactured by the Nisshin OilliO Group Ltd. and sold under a trade name of "Nisshin safflower oil"), and corn oil (manufactured by the Nisshin OilliO Group Ltd. and sold under a trade name of "Nisshin corn oil").

### [Manufacturing method of flax oil]

After flax seeds (produced in Canada) were heated to **80** degrees C, an expression treatment was applied so as to obtain flax crude oil, and **3** mass% of water to the crude oil was added at **70** degrees C while being stirred and maintained for **20** minutes, and then, a centrifugal separation was carried out at **3000**×G for **20** minutes so as to precipitate and eliminate water dispersible phospholipid, and clear upper portion obtained was dried by being heated to **100** degrees C under reduced pressure by using a vacuum pump, so as to obtain degumming oil. Next, **0.1** mass% of phosphoric acid having a concentration of **75** volume% to the degumming oil was added to the degumming oil, after being maintained for **15** minutes while continuously being stirred at **70** degrees C, acid value was measured, next, an aqueous solution of sodium hydroxide having Baume degree of **16** was added in a **50**% excessive amount than an amount needed for carrying out saponification of free fatty acid, after being maintained for **15** minutes while continuously being stirred at 70 degrees C, a centrifugal separation was carried out at **3000**×G for **20** minutes, and after a mixture of non-water dispersible phospholipid and fatty acid soap was precipitated and eliminated as oil cake, water washing was repeated until water washing liquid is neutralized, so as to carry out deacidification treatment. After that, drying was carried out by being heated to **100** degrees C under reduced pressure by using a vacuum pump. Subsequently, after **1** mass% of activated white earth (manufactured by Mizusawa Chemicals, Ltd. to the dried oil was added to the dried oil and maintained at **110** degrees C for **20** minutes under reduced pressure by using a vacuum pump, the white earth was separated by filtration at **85** degrees C, so as to carry out decolorization treatment. Further, steam blowing deodorization treatment (total amount of steam blowing: **5** mass% to oil) was carried out at **230** degrees C for **60** minutes, so as to fabricate purified flax oil having the α-linolenic acid content of **56.3** mass% and the trans fatty acid content of **2.5** mass%.

With regard to each of the obtained oil-and-fat compositions of Examples **1** to **9** and Comparative Examples **1** to **4,** evaluations of flavor and cooked odor were carried out according to the evaluation test method described below. Evaluation results are shown in Tables **1** to **3.**

### [Evaluation of flavor]

The evaluation of flavor was carried out by that a panel of seven experts put almost **1** to **2** ml of the oil-and-fat composition in a state of normal temperature into their mouth, so as to evaluate according to the criterion for evaluation (on a scale of **1** to **5**) described below, and the average value was determined as the evaluation value.

### (criterion for evaluation)

Scale of **5:** very good
Scale of **4:** goodish
Scale of **3:** common
Scale of **2:** baddish
Scale of **1:** very bad

### [Evaluation of heated odor (cooked odor)]

The evaluation of heated odor(cooked odor) was carried out by that 50g of the oil-and-fat composition were fed in a beaker of **100** ml in volume and heated to **120** degrees C, and a panel of seven experts evaluated according to the criterion for evaluation (on a scale of **1** to **5**) described below, and the average value was determined as the evaluation value.

### (criterion for evaluation)

Scale of **5**: very good
Scale of **4:** goodish
Scale of **3:** common
Scale of **2:** baddish
Scale of **1:** very bad

Further, with regard to each of the obtained oil-and-fat compositions, storage test was carried out according to the storage test method described below. Evaluation results are shown in Tables **1** to **3.**

### [Evaluation of storage stability]

The evaluation of storage stability was carried out by that the oil-and-fat composition was fed in a PET container of **600** g in volume, and stored at the storing temperature of **40** degrees C for **12** weeks and stored at the storing temperature of **20** degrees C and under the illuminance of **1000** lux for **12** weeks, and then flavor and cooked odor were evaluated as well as the above description.

[Table **1**]

**Table 1**

| | | Example 1 | Example 2 | Example 3 | Example 4 |
|---|---|---|---|---|---|
| Blending oil and Blending ratio | | Flax/Rice/Rapeseed **=20/20/60** | Flax/Rice/Rapeseed **=25/20/55** | Flax/Rice/Rapeseed **=30/20/50** | Flax/Rice/Rapeseed **=35/20/45** |
| α-linolenic content acid | | **17.3%** | **19.6%** | **21.8%** | **24.1%** |
| Before storage | Flavor | **4.6** | **4.7** | **4.6** | **4.6** |
| | Cooked odor | **4.6** | **4.4** | **4.6** | **4.6** |
| After storage 1 | Flavor | **4.1** | **4.0** | **4.1** | **4.0** |
| | Cooked odor | **4.0** | **4.1** | **4.0** | **4.1** |
| After storage 2 | Flavor | **4.1** | **4.1** | **4.1** | **4.0** |
| | Cooked odor | **4.0** | **4.0** | **4.1** | **4.1** |

| | | | | | |
|---|---|---|---|---|---|
| (Notes) After storage **1**: after stored at 40 degrees C for 12 weeks After storage **2**: after stored at 20 degrees C and under 1000 lux for 12 weeks | | | | | |

[Table **2**]

**Table 2**

| | | Example 5 | Example 6 | Example 7 | Example 8 | Example 9 |
|---|---|---|---|---|---|---|
| Blending oil and Blending ratio | | Flax/Rice/Rapeseed **=35/15/50** | Flax/Rice/Rapeseed **=25/15/60** | Flax/Rice/Rapeseed **=25/25/50** | Flax/Rice **=30/70** | Flax/Rice **=50/50** |
| α-linolenic acid content | | **24.6%** | **20.1%** | **19.1%** | **16.8%** | **27.8%** |
| Before storage | Flavor | **4.4** | **4.7** | **4.7** | **4.7** | **4.7** |
| | Cooked odor | **4.4** | **4.3** | **4.9** | **4.7** | **4.6** |
| After storage **1** | Flavor | **4.0** | **4.3** | **4.1** | **4.1** | **4.3** |
| | Cooked odor | **4.0** | **4.0** | **4.6** | **4.3** | **4.1** |
| After storage **2** | Flavor | **4.0** | **4.0** | **4.3** | **4.3** | **4.1** |
| | Cooked odor | **4.0** | **4.0** | **4.0** | **4.1** | **4.1** |

| | | | | | | |
|---|---|---|---|---|---|---|
| (Notes) After storage **1**: after stored at 40 degrees C for 12 weeks After storage **2**: after stored at 20 degrees C and under 1000 lux for 12 weeks | | | | | | |

[Table **3**]

**Table 3**

| | | Com. Ex. 1 | Com. Ex. 2 | Com. Ex. 3 | Com. Ex. 4 | Com. Ex. 5 | Com. Ex. 6 |
|---|---|---|---|---|---|---|---|
| Blending oil and Blending ratio | | Flax/Rice/Rapeseed **=50/10/40** | Flax /Rapeseed **=50/50** | Flax/High-oleic safflower oil **=60/40** | Flax/Corn/ Rapeseed **=20/60/20** | Flax/Corn/ Rapeseed **=30/30/40** | Flax/Rice/Rapeseed **=40/20/40** |
| α-linolenic acid content | | **31.8%** | **32.5%** | **33.1%** | **13.2%** | **20.1%** | **26.4** |
| Before storage | Flavor | **3.6** | **3.4** | **3.4** | **4.0** | **3.7** | **3.7** |
| | Cooked odor | **3.7** | **3.3** | **3.4** | **3.9** | **3.9** | **3.7** |
| After storage 1 | Flavor | **2.6** | **2.4** | **2.9** | **3.3** | **3.1** | **3.1** |
| | Cooked odor | **2.7** | **2.1** | **2.7** | **3.4** | **3.1** | **3.3** |
| After storage 2 | Flavor | **2.1** | **2.0** | **2.4** | **3.1** | **3.1** | **3.1** |

| | | | | | | | |
|---|---|---|---|---|---|---|---|
| (Notes) Com. Ex.: Comparative Example After storage 1: after stored at 40 degrees C for 12 weeks After storage 2: after stored at 20 degrees C and under 1000 lux for 12 weeks | | | | | | | |

Further, with regard to each of the obtained oil-and-fat compositions of Examples **1** to **9** and Comparative Examples **1** to **4,** cooking test was carried out according to the cooking test method described below. Evaluation results are shown in Tables **4** to **6.**

### [Evaluation of cooking]

Each of the food items described in Tables **4** to **6** was cooked in the usual manner, odor at the cooking and flavor at the eating were evaluated. The evaluation was carried out by a panel of seven experts according to the criterion for evaluation (on a scale of **1** to **5**) described below, and the average value was determined as the evaluation value.

### (criterion for evaluation)

Scale of **5:** very good
Scale of **4:** goodish
Scale of **3:** common
Scale of **2:** baddish
Scale of **1**: very bad

[Table **4**]

**Table 4**

| | Example 1 | Example 2 | Example 3 | Example 4 |
|---|---|---|---|---|
| Blending oil and Blending ratio | Flax/Rice/Rapeseed =**20/20/60** | Flax/Rice/Rapeseed =**25/20/55** | Flax/Rice/Rapeseed =**30/20/50** | Flax/Rice/Rapeseed =**35/20/45** |
| Deep-fried sweet potato | **4.7** | **4.7** | **4.9** | **4.4** |
| Deep-fried shrimp | **4.7** | **4.6** | **4.6** | **4.6** |
| Croquette | **4.6** | **4.6** | **4.9** | **4.6** |
| Fried chicken | **4.6** | **4.6** | **4.6** | **4.6** |
| Scramble eggs | **4.7** | **4.7** | **4.4** | **4.6** |
| Stir-fried vegetables | **4.6** | **4.7** | **4.6** | **4.4** |
| Dressing | **4.6** | **4.6** | **4.4** | **4.6** |

[Table **5**]

**Table 5**

| | Example 5 | Example 6 | Example 7 | Example 8 | Example 9 |
|---|---|---|---|---|---|
| Blending oil and Blending ratio | Flax/Rice/Rapeseed **=35/15/50** | Flax/Rice/Rapeseed **=25/15/60** | Flax/Rice/Rapeseed **=25/25/50** | Flax/Rice **=30/70** | Flax/Rice **=50/50** |
| Deep-fried sweet potato | **4.2** | **4.3** | **4.7** | **4.9** | **4.4** |
| Deep-fried shrimp | **4.4** | **4.6** | **4.7** | **4.6** | **4.7** |
| Croquette | **4.4** | **4.6** | **4.4** | **4.7** | **4.6** |
| Fried chicken | **4.2** | **4.6** | **4.6** | **4.6** | **4.6** |
| Scramble eggs | **4.4** | **4.4** | **4.6** | **4.6** | **4.6** |
| Stir-fried vegetables | **4.4** | **4.4** | **4.6** | **4.7** | **4.6** |
| Dressing | **4.4** | **4.6** | **4.7** | **4.6** | **4.6** |

[Table **6**]

**Table 6**

| | Com. Ex. 1 | Com. Ex. 2 | Com. Ex. 3 | Com. Ex. 4 | Com. Ex. 5 | Com. Ex. 6 |
|---|---|---|---|---|---|---|
| Blending oil and Blending ratio | Flax/Rice/Rapeseed **=50/10/40** | Flax/Rapeseed **=50/50** | Flax/High-oleic safflower oil **=60/40** | Flax/Corn/Rapeseed **=20/60/20** | Flax/Corn/Rapeseed **=30/30/40** | Flax/Rice/Rapeseed **=40/20/40** |
| Deep-fried sweet potato | **3.4** | **3.1** | **3.3** | **3.6** | **3.3** | **3.4** |
| Deep-fried shrimp | **3.6** | **3.0** | **3.3** | **3.4** | **3.4** | **3.4** |
| Croquette | **3.1** | **2.8** | **3.1** | **3.7** | **3.1** | **3.3** |
| Fried chicken | **3.1** | **3.1** | **3.0** | **3.4** | **3.0** | **3.1** |
| Scramble eggs | **3.1** | **3.1** | **3.1** | **3.7** | **3.4** | **3.6** |
| Stir-fried vegetables | **3.0** | **3.3** | **3.1** | **3.4** | **3.4** | **3.4** |
| Dressing | **3.4** | **3.3** | **3.4** | **3.6** | **3.6** | **3.4** |

| | | | | | | |
|---|---|---|---|---|---|---|
| (Notes) Com. Ex.: Comparative Example | | | | | | |

As is clear from Tables **1** to **6**, the oil-and-fat compositions of Examples **1** to **9** according to the invention have a good flavor before and after the storage and do not have an unpleasant odor at the heating, and further, have a good odor at the cooking and a good flavor at the eating.

On the other hand, it is known that the oil-and-fat compositions of the Comparative Examples **1** to **4** of which blending component, blending ratio or α-linolenic acid content is out of the range defined in the present invention are inferior in any of flavor and cooked odor before and after the storage, and odor at the cooking and flavor at the eating to those of Examples **1** to **9.**

### (Example 10, Comparative Example 5, Reference Example 1)

Next, the hypertension-ameliorating effect verification test described below was carried out by using the above-mentioned oil-and-fat composition of Example **2** (Example **10**), salad oil moderately containing α-linolenic acid (Comparative Example **5**) and high-oleic safflower oil (manufactured by the Nisshin OilliO Group Ltd. and sold under a trade name of "Nisshin safflower oil") (Reference Example **1**) as test oils. The high-oleic safflower oil contains very little α-linolenic acid so that it is used as a reference oil. As the salad oil moderately containing α-linolenic acid, a mixture of salad oil (manufactured by the Nisshin OilliO Group Ltd. and sold under a trade name of "Nisshin soybean salad oil (S)") and salad oil (manufactured by the Nisshin OilliO Group Ltd. and sold under a trade name of "Nisshin rape seed salad oil (S)") having a mixing ratio of **1** : **1** was used for the test.

### [Hypertension-ameliorating effect verification test by a forced oral administration to rats]

As a test animal, **5**-week-old and male SHR/Izm being a hypertension pathogenesis model rat was used, and after a preliminary breeding of **6** days, **1** ml (**0.92** g) of the test oil was forcibly and orally administered by using a stomach sound. After **4** hours from the forced oral administration, a cuff was wrapped around the tail portion of the rat, and the blood pressure was measured by using a non-invasive blood pressure monitor (manufactured by Muromachi Kikai Co., Ltd. and sold under a model number of "MK-**2000**"). As the feeding stuff during the preliminary breeding and test, solid feed (manufactured by PMI Nutrition International and sold under a trade name of "PicoLab Rodent Diet **20**") was freely consumed and drinkable water was also freely consumed. The blood pressure of the rat by which each of the test oils was consumed after **4** hours from the forced oral administration are shown in Table **7**. Further, the fatty acid composition of each of the test oils is shown in Table **8**.

[Table 7]

**Table 7**

| | Test oil | Blood pressure after 4 hours from forced oral administration (mmHg) |
|---|---|---|
| Example 10 | Oil-and-fat composition of Example 2 | **142.1±9.6b** |
| Com. Ex. 5 | Salad oil | **159.4 ± 13.4a** |
| Ref. Ex. 1 | High-oleic safflower oil | **167.2±8.1a** |

| | | |
|---|---|---|
| (Notes) Com. Ex.: Comparative Example Ref. Ex.: Reference Example | | |

The blood pressure after **4** hours from the forced oral administration were shown by the average value ± standard deviation. Between groups having different alphabets (a, b), statistically significant differences (P<**0.05**) were seen. Statistical processing was carried out by Bonferroni multiple comparison calibration.

As is clear from Table **7**, it was verified that the oil-and-fat composition of Example **2** (Example **10**) has a hypertension-ameliorating effect on rats, since the blood pressure of rat in case of Example **10** was significantly decreased in comparison with the blood pressures in cased of the salad oil (Comparative Example **5**) and the high-oleic safflower oil (Reference Example **1**).

On the other hand, the salad oil moderately containing α-linolenic acid (Comparative Example **5**) has a tendency that the blood pressure is decreased in comparison with the high-oleic safflower oil (Reference Example **1**), but no significant differences are recognized.

[Table **8**]

**Table 8**

| Fatty acid composition of test oil (%) | | | |
|---|---|---|---|
| | Example 10 | Com. Ex. 5 | Ref. Ex. 1 |
| **16 : 0** | **6.9** | **7.5** | **4.9** |
| **18 : 0** | **2.1** | **2.9** | **2.0** |
| **18 : 1n-9** | **46.0** | **41.3** | **77.5** |
| **18 : 2n-6** | **22.3** | **36.9** | **13.7** |
| **18 : 3n-3** | **19.9** | **8.7** | **0.6** |
| **20 : 0** | **0.5** | **0.5** | **0** |
| **20 : 1n-9** | **0.8** | **0.7** | **0.4** |
| **22 : 0** | **0.2** | **0.4** | **0.3** |
| **24 : 0** | **0.1** | **0.2** | **0.2** |
| **Others** | **1.2** | **0.9** | **0.4** |

| | | | |
|---|---|---|---|
| (Notes) Com. Ex.: Comparative Example Ref. Ex.: Reference Example | | | |

## Claims

1. An oil-and-fat composition, comprising either
i. 20-35 mass% of at least one vegetable oil A selected from flax oil, perilla oil and perilla seed oil; and
15-25 mass% of rice oil, or
ii. 30-53 mass% of at least one vegetable oil A selected from flax oil, perilla oil and perilla seed oil; and
70-47 mass% of rice oil,
wherein in the case of both (i) and (ii), the α-linolenic acid content in the constituent fatty acids contained in the oil-and-fat compositions is 15 to 30 mass%.

2. An oil-and-fat composition according to claim 1, comprising:
at least one vegetable oil A selected from flax oil, perilla oil and perilla seed oil;
rice oil; and
at least one vegetable oil B selected from rape seed oil, soybean oil and corn oil, at a ratio among the vegetable oil A, the rice oil and the vegetable oil B of 20-35/15-25/40-65 by mass, wherein:
the α-linolenic acid content in the constituent fatty acids contained in the oil-and-fat compositions is 15 to 30 mass%

3. The oil-and-fat composition according to claim 1 or claim 2, wherein:
the vegetable oil A is flax oil.

4. The oil-and-fat composition according to claim 2 or 3 as dependant therefrom, wherein:
the vegetable oil B is rape seed oil.

5. The oil-and-fat composition according to any one of claims 1 to 4, wherein:
the trans fatty acid content in the oil-and-fat composition is not more than 3 mass%.

6. A food or beverage containing the oil-and-fat composition according to any one of claims 1 to 5.

## Patentansprüche

1. ÖI-und-Fett-Zusammensetzung, die entweder:
i. 20 bis 35 Massen-% zumindest eines aus Flachsöl, Perillaöl und Perillasamenöl ausgewählten Pflanzenöls A und
15 bis 25 Massen-% Reisöl; oder
ii. 30 bis 53 Massen-% zumindest eines aus Flachsöl, Perillaöl und Perillasamenöl ausgewählten Pflanzenöls A und
70 bis 47 Massen-% Reisöl;
umfasst, worin sowohl im Falle von (i) als auch von (ii) der α-Linolensäuregehalt in den einzelnen Fettsäuren, die in der ÖI-und-Fett-Zusammensetzung enthalten sind, 15 bis 30 Massen-% beträgt.

2. Öl-und-Fett-Zusammensetzung nach Anspruch 1, die Folgendes umfasst:
zumindest ein aus Flachsöl, Perillaöl und Perillasamenöl ausgewähltes Pflanzenöl A;
Reisöl; und
zumindest ein aus Rapsöl, Sojaöl und Maisöl ausgewähltes Pflanzenöl B in einem Verhältnis von 20-35 zu 15-25 zu 40-65 Massen-% zwischen Pflanzenöl A, Reisöl und Pflanzenöl B, worin:
der α-Linolensäuregehalt in den einzelnen Fettsäuren, die in der Öl-und-Fett-Zusammensetzung enthalten sind, 15 bis 30 Massen-% beträgt.

3. ÖI-und-Fett-Zusammensetzung nach Anspruch 1 oder Anspruch 2, worin:
das Pflanzenöl A Flachsöl ist.

4. ÖI-und-Fett-Zusammensetzung nach Anspruch 2 oder dem davon abhängigen Anspruch 3, worin:
das Pflanzenöl B Rapsöl ist.

5. ÖI-und-Fett-Zusammensetzung nach einem der Ansprüche 1 bis 4, worin:
der Trans-Fettsäure-Gehalt in der Öl-und-Fett-Zusammensetzung nicht mehr als 3 Massen-% beträgt.

6. Nahrungsmittel oder Getränk, das eine ÖI-und-Fett-Zusammensetzung nach einem der Ansprüche 1 bis 5 enthält.

## Revendications

1. Composition d'huile et de graisse, comprenant l'un de
i. de 20 à 35 % en masse d'au moins une huile végétale A choisie parmi l'huile de lin, l'huile de périlla et l'huile de graines de périlla ; et
de 15 à 25 % en masse d'huile de riz, ou
ii. de 30 à 53 % en masse d'au moins une huile végétale A choisie parmi l'huile de lin, l'huile de périlla et l'huile de graines de périlla ; et
de 70 à 47 % en masse d'huile de riz,
où, dans le cas de (i) et (ii), la teneur en acide α-linolénique dans les acides gras constituants contenus dans les compositions d'huile et de graisse est de 15 à 30 % en masse.

2. Composition d'huile et de graisse selon la revendication 1, comprenant :
au moins une huile végétale A choisie parmi l'huile de lin, l'huile de périlla et l'huile de graines de périlla ;
de l'huile de riz ; et
au moins une huile végétale B choisie parmi l'huile de colza, l'huile de soja et l'huile de maïs, à un rapport entre l'huile végétale A, l'huile de riz et l'huile végétale B de 20-35/15 à 25/40-65 en masse, où :
la teneur en acide α-linolénique dans les acides gras constituants contenus dans les compositions d'huile et de graisse est de 15 à 30 % en masse.

3. Composition d'huile et de graisse selon la revendication 1 ou la revendication 2, dans laquelle :
l'huile végétale A est l'huile de lin.

4. Composition d'huile et de graisse selon la revendication 2 ou 3 dépendante de celle-ci, dans laquelle :
l'huile végétale B est l'huile de colza.

5. Composition d'huile et de graisse selon l'une quelconque des revendications 1 à 4, dans laquelle :
la teneur en acides gras trans dans la composition d'huile et de graisse est de pas plus de 3 % en masse.

6. Aliment ou boisson contenant la composition d'huile et de graisse selon l'une quelconque des revendications 1 à 5.
